# EUROPEAN PATENT SPECIFICATION

(11) **EP 1 800 608 B1**
(45) Date of publication and mention of the grant of the patent: **05.09.2012**
(21) Application number: 06256429.9
(22) Date of filing: 19.12.2006
(51) Int. Cl.: A61F 2/08, A61B 17/04

(54) **Materials for ligament reconstruction**
Materialien zur Ligamentrekonstruktion
Matériaux de reconstruction ligamentaire

(30) Priority: 20.12.2005 US 306239
(43) Date of publication of application: 27.06.2007
(73) Proprietor: DePuy Mitek, Inc., Raynham, MA 02767 (US)
(72) Inventor: Whittaker, Gregory R, Stoneham, MA 02180 (US); Hull, Les, Attleboro, Massachusetts 02703 (US)
(74) Representative: Fisher, Adrian John

(56) References cited:
- EP-A- 1 481 651
- EP-A2- 1 180 351
- WO-A-94/28799
- WO-A-99/51159
- US-A1- 2002 156 476
- US-A1- 2005 273 003
- US-B1- 6 325 804
- US-B1- 6 652 592

## Description

### FIELD OF THE INVENTION

The present invention relates to devices for affixing ligament grafts in bone tunnels.

### BACKGROUND OF THE INVENTION

Joint injuries may commonly result in the complete or partial detachment of ligaments, tendons and soft tissues from bone. Tissue detachment may occur in many ways, e.g., as the result of an accident such as a fall, overexertion during a work-related activity, during the course of an athletic event, or in any one of many other situations and/or activities. These types of injuries are generally the result of excess stress or extraordinary forces being placed upon the tissues.

In the case of a partial detachment, commonly referred to under the general term "sprain," the injury frequently heals without medical intervention, the patient rests, and care is taken not to expose the injury to undue strenuous activities during the healing process. If, however, the ligament or tendon is completely detached from its attachment site on an associated bone or bones, or if it is severed as the result of a traumatic injury, surgical intervention may be necessary to restore full function to the injured joint. A number of conventional surgical procedures exist for re-attaching such tendons and ligaments to bone.

One such procedure involves the re-attachment of the detached tissue using "traditional" attachment devices such as staples, sutures, and bone screws. Such traditional attachment devices have also been used to attach tendon or ligament grafts (often formed from autologous tissue harvested from elsewhere in the body) to the desired bone or bones. In one procedure, a damaged anterior cruciate ligament ("ACL") is replaced in a human knee. Initially bone tunnels are formed through the tibia and femur at the points of normal attachment of the anterior cruciate ligament. Next, a ligament graft with a bone graft on one of its ends is sized so as to fit within the bone tunnels. Suture is then attached to the bone graft and thereafter passed through the tibia and femoral bone tunnels. The bone graft is then pulled through the tibial tunnel and up into the femoral tunnel using the suture. As this is done, the ligament graft ligament extends back out of the femoral tunnel, across the interior of the knee joint, and then through the tibial tunnel. The free end of the ligament graft ligament resides outside the tibia, at the anterior side of the tibia. Next, a bone screw is inserted between the bone graft and the wall of femoral bone tunnel so as to securely lock the bone graft in position by a tight interference fit. Finally, the free end of the ligament graft ligament is securely attached to the tibia.

WO 94/28799 discusses an anchor for attaching an object within a tunnel and a method for using the same. The anchor includes a body, and a plurality of barbs located in circumferentially spaced relation about the body.

US 2002/156476 discusses a graft fixation device comprising a threaded body which is rotatably connected to a graft interface member which can include an enclosed loop for holding a soft tissue graft.

In another ACL reconstruction procedure, aligned femoral and tibial tunnels are initially formed in a human knee. A bone graft with a ligament graft attached thereto is passed through the tunnels to a blind end of the femoral tunnel where the block is fixed in place by an anchor. The ligament extends out of the tibial tunnel, and the end is attached to the tibia cortex by staples or the like. Alternatively, the end of the ligament may be fixed in the tibial tunnel by an anchor or by an interference screw. Various types of ligament and/or suture anchors for attaching soft tissue to bone are also well known in the art. A number of these devices are described in detail in U.S. Pat. Nos. 4,898,156, 4,899,743, 4,968,315, 5,356,413, and 5,372,599, all of which are commonly assigned to Mitek Surgical Products, Inc., a Johnson & Johnson company.

One known method for anchoring bone grafts in bone tunnels is through a "cross-pinning" technique, in which a pin, screw, or rod is driven into the bone transversely to the bone tunnel so as to intersect the bone graft and thereby cross-pin the bone graft in the bone tunnel. In order to provide for proper cross-pinning of the bone graft in the bone tunnel, a drill guide is generally used. The drill guide serves to ensure that the transverse passage is positioned in the bone so that it will intersect the appropriate tunnel section and the bone graft.

EP 1180351 A and US 2005/0273003 discuss devices and methods according to this technique. The preamble of appended claim 1 is based on EP 1 180 351 A.

While cross-pinning is effective, there is a continuing need for improved methods and devices for fixing a ligament graft in a bone tunnel.

### SUMMARY OF THE INVENTION

The present invention generally provides a device, as claimed in claim 1, for fixing a ligament graft in a bone tunnel. The device may be used in a method which includes drilling a first tunnel in bone and inserting a ligament graft at least partially into the first tunnel. An adhesive is introduced into the first tunnel, and a cross-pin is inserted through the ligament graft in a direction substantially transverse to an axis of the tunnel to maintain the ligament graft in contact with the adhesive. The cross-pin can be removed when the adhesive is cured such that the ligament graft is fixed in the first tunnel.

While the device can be used in a variety of surgical procedures, in one example the device is used to repair an anterior cruciate ligament. Thus, the ligament graft can be at least partially inserted into a femoral or tibial tunnel formed in a femur or tibia, and the adhesive is introduced into the femoral or tibial tunnel. A cross-pin, is inserted through a transverse tunnel formed in the femur or tibia and intersecting the femoral or tibial tunnel. In certain methods, the transverse tunnel can include a sleeve disposed therein and defining a pathway to the femoral or tibial tunnel. The cross-pin is effective to maintain a first end of the ligament graft in the femoral or tibial tunnel in contact with the adhesive. A second end of the ligament graft can then be tensioned and fixed in the other one of the femoral or tibial tunnel, while the adhesive is drying. Once the adhesive is cured, the fixation device can be removed.

The first tunnel can have a variety of configurations, and it can extend only partially through the femur or tibial, or fully through the femur or tibia. In one example, the first tunnel includes an end wall formed therein. The transverse tunnel can intersect the first tunnel adjacent the end wall. The adhesive can thus be introduced through the transverse tunnel such that the adhesive is disposed adjacent to the end wall of the first tunnel. In one example, the ligament graft is positioned a distance apart from the end wall of the first tunnel prior to introducing the adhesive, and it is pulled toward the end wall and into contact with the adhesive after the adhesive is introduced into the first tunnel. The ligament graft can optionally be pulled through the first tunnel using a suture attached thereto.

In other examples, the adhesive can be formed from a bioabsorbable material, such that it is eventually absorbed by the body. Alternatively, the adhesive can be a non-absorbable adhesive. In accordance with the invention, the cross-pin is adapted to prevent adhesion between the adhesive and the cross-pin. For example, the cross-pin can include a protective coating disposed thereon, or it can be formed from a protective material, such as a fluoropolymer plastic resin. The sleeve disposed within the transverse tunnel can also include a protecting coating, or it can be formed from a protective material.

. The anchor has a first portion that is adhered to the bone tunnel, and a second portion that mates to the bone graft. The first portion can include a post with an eyelet formed on a terminal end thereof. In order to introduce the anchor and bone graft into a bone tunnel, a suture can be attached to the eyelet and tensioned the suture to pull the bone graft into the bone tunnel. The second portion of the anchor can also have a variety of configurations. For example, it can include threads formed thereon for threading the second portion into the bone graft. In another embodiment, the second portion can be mated to the bone graft using sutures. In particular, the second portion can include opposed arms, and the bone graft can be mated to the opposed arms by passing at least one suture through the opposed arms and through the bone graft.

### BRIEF DESCRIPTION OF THE DRAWINGS

The invention will be more fully understood from the following detailed description taken in conjunction with the accompanying drawings, in which:
FIG. 1 illustrates a human knee and a guide device having a cannulated guide rod extending through bone tunnels formed in the tibia and femur;
FIG. 2 illustrates the human knee and guide device of FIG. 1, showing a sleeve and trocar assembly being inserted through the femur to form a transverse tunnel that intersects the femoral tunnel;
FIG. 3 illustrates the human knee of FIG. 2 with the guide device and the trocar removed, leaving the sleeve extending into the transverse tunnel, and a ligament graft extending through the tibial tunnel and into the femoral tunnel;
FIG. 4 illustrates the human knee of FIG. 3, showing a syringe inserted through the sleeve in the transverse tunnel and injecting an adhesive into the femoral tunnel;
FIG. 5 illustrates the human knee of FIG. 4, showing the ligament graft pulled further into the femoral tunnel and into contact with the adhesive such that the adhesive surrounds an end of the ligament graft;
FIG. 6 illustrates the human knee of FIG. 5, showing a cross-pin being inserted through the sleeve, into the transverse tunnel, and through the ligament graft to maintain the ligament graft in a fixed position within the femoral tunnel;
FIG. 7 illustrates the human knee of FIG. 6 showing the sleeve removed leaving the cross-pin in place;
FIG. 8 illustrates the human knee of FIG. 7 showing the cross-pin removed after the adhesive has cured;
FIG. 9A is a side perspective view of one exemplary embodiment of an anchor configured to mate to a bone graft and to be affixed within a bone tunnel;
FIG. 9B is a side perspective view of the anchor shown in FIG. 9A mated to a bone graft with a ligament graft extending therefrom;
FIG. 9C is an illustration showing the anchor, bone graft, and ligament graft of FIG. 9B implanted within a bone tunnel in bone, showing space in the bone tunnel for receiving an adhesive;
FIG. 10 is a side perspective view of another embodiment of an anchor configured to mate to a bone graft and to be affixed within a bone tunnel to anchor the bone graft within the bone tunnel, the anchor having annual grooves for receiving an adhesive;
FIG. 11A is a side view of another embodiment of an anchor and a bone graft, each having suture bores formed therein for mating the bone graft to the anchor;
FIG. 11B is another side view of the anchor and bone graft shown in FIG. 11A;
FIG. 11C is a side view of the anchor and bone graft shown in FIGS. 11A and 11B mated to one another using sutures;

### DETAILED DESCRIPTION OF THE INVENTION

The present invention provides devices for fixing a ligament graft in a bone tunnel. The various devices disclosed herein can be used in a variety of surgical procedures, however the methods and devices are particularly useful for repairing an anterior cruciate ligament (ACL) in a human knee. In an ACL repair, the tom ACL is replaced with a ligament graft which is anchored to the tibia and femur. The term "ligament graft," as used herein, is intended to include natural materials, such as autografts, allografts, and xenografts, including harvested ligaments and tendons, as well as synthetic materials. The ligament graft can also include an anchoring element attached thereto for anchoring the graft to the tibia and femur. For example, the ligament graft can include a bone graft, plug, or other member, attached to one or both terminal ends thereof. The term "bone graft," as used herein, in intended to include natural materials, such as autografts, allografts, and xenografts, as well as synthetic materials.

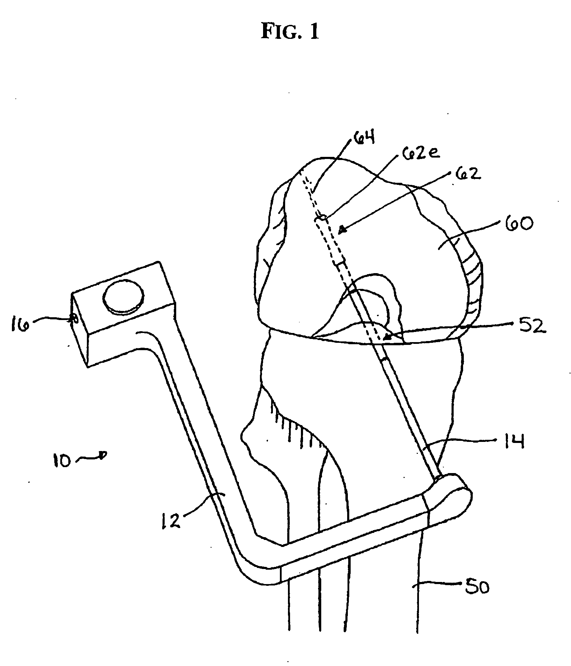

FIGS. 1-8 illustrate one exemplary method for anchoring a ligament graft in a bone tunnel, and in particular in femoral and tibial tunnels of a human knee. In general, the method includes drilling a tunnel in bone and inserting a ligament graft at least partially into the tunnel. An adhesive is introduced into the tunnel, and a fixation device, in the form of a cross-pin, is inserted through the ligament graft in a direction transverse to an axis of the tunnel to maintain the ligament graft in contact with the adhesive. The fixation device can be removed when the adhesive is cured such that the ligament graft is fixed in the tunnel. The use of a fixation device to maintain the ligament graft in the tunnel while the adhesive cures allows an opposed end of the ligament graft to be tensioned and fixed using the same or a different anchoring technique. The method is described in connection with certain exemplary procedures for preparing bone tunnels and inserting a ligament graft into the bone tunnels. For example, in certain exemplary methods, the ligament graft is affixed in the femoral tunnel prior to affixing it in the tibial tunnel. However, a person skilled in the art will appreciate that the ligament graft can be affixed in the tibial tunnel first or at the same time. A person skilled in the art will also appreciate that a variety of other procedures known in the art can be used to prepare the bone tunnels and to the insert a ligament graft into the bone tunnels.

Referring first to FIG. 1, a bone tunnel is drilled through the tibia 50 and femur 60, using conventional surgical equipment and techniques, to form a tibial tunnel 52 and femoral tunnel 62. The tibial and femoral tunnels 52, 62 can extend completely through the tibia and femur 50, 60, however in an exemplary embodiment the femoral tunnel 62 terminates part way through the femur 60 such that a femoral socket is formed. A suture tunnel 64 can optionally extend through the remainder of the femur 60 in longitudinal alignment with the femoral tunnel 62 to allow a suture to pass therethrough and pull and graft into the tunnels 52, 62, as will be discussed in more detail below. The suture tunnel 64 preferably has an inner diameter that is less than an inner diameter of the femoral tunnel, such that a terminal end of the femoral tunnel defines an end wall 62e.

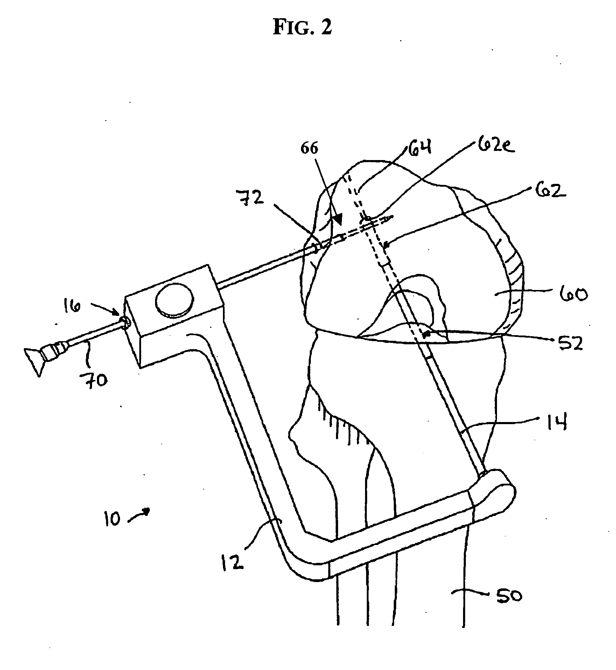

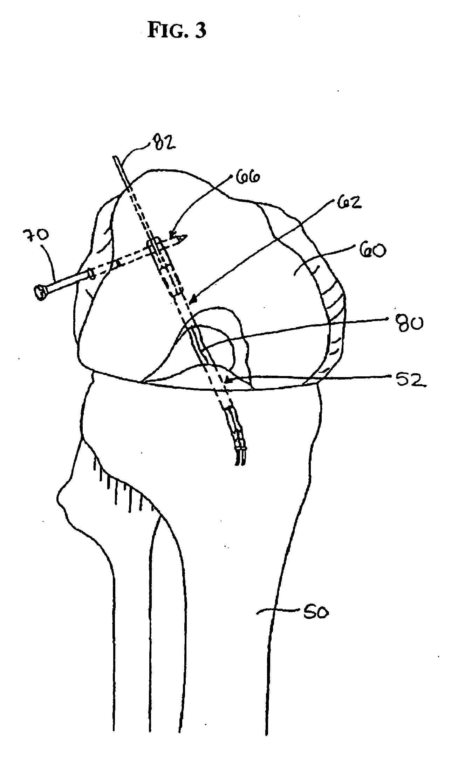

Once the tibial and femoral tunnels 52, 62 are prepared, a transverse tunnel can be formed in the femur. The transverse tunnel preferably intersects the femoral tunnel 62 such that a pathway is formed through the transverse tunnel to the femoral tunnel 62. This pathway can be used to inject an adhesive into the femoral tunnel 62, as will be discussed in more detail below. As shown in FIG. 1, a guide device 10 can optionally be used to locate and align the transverse tunnel. As shown, the guide device 10 generally includes an L-shaped frame 12 having a cannulated guide rod 14 extending from one end thereof, and a housing with an opening 16 formed therethrough on an opposed end thereof. The cannulated guide rod 14 can be inserted through the tibial tunnel 52 and the femoral tunnel 62 to maintain the guide frame 12 in a fixed position. As shown in FIG. 2, a sleeve and trocar assembly 70, 72 can then be inserted through the opening 16 in the housing on the guide frame 12, and drilled into the lateral side of the femur until the sleeve and trocar abut the cannulated guide rod 14. The transverse tunnel 66 will thus extend through the femur and into the femoral tunnel 62, preferably at a location substantially adjacent to the distal end wall 62e of the femoral tunnel. A person skilled in the art will appreciate that the transverse tunnel 66 can extend at any angle relative to the femoral tunnel 62. After the transverse tunnel 66 is completely formed, the trocar 72 can be removed leaving the sleeve 70 in place, as shown in FIG. 3. The sleeve 70 can be inserted partially into the transverse tunnel 66, such that it does not extend into the femoral tunnel 62, or alternatively the sleeve 70 can at least partially extend into the femoral tunnel 62. This will allow the sleeve 70 to function as a stop for the ligament graft, as will be discussed in more detail below. The procedure can, however, be performed without the use of the sleeve 70.

After the tibial, femoral, and transverse tunnels 52, 62, 66 are formed, a ligament graft 80 can be introduced into the tibial and femoral tunnels 52, 62. While various procedures known in the art can be used to introduce the ligament graft 80 into the tibial and femoral tunnels 52, 62, in one exemplary embodiment a suture 82 can be attached to a leading end of the ligament graft 80 and it can be threaded through the tibial and femoral tunnels 52, 62 using, for example, a guide pin or other device. Tension can then be applied to the suture to pull the ligament graft 80 up through the tibial tunnel 52 and at least partially into the femoral tunnel 62. As shown in FIG. 3, the ligament graft 80 can be pulled to a location that is spaced a distance apart from the end wall 62e of the femoral tunnel, such that the ligament graft 80 stops short of the transverse tunnel 66. Where the sleeve 70 extends into the femoral tunnel 62, the sleeve 70 will prevent the ligament graft 80 from being fully advanced into contact with the end surface 62e of the femoral tunnel 62.

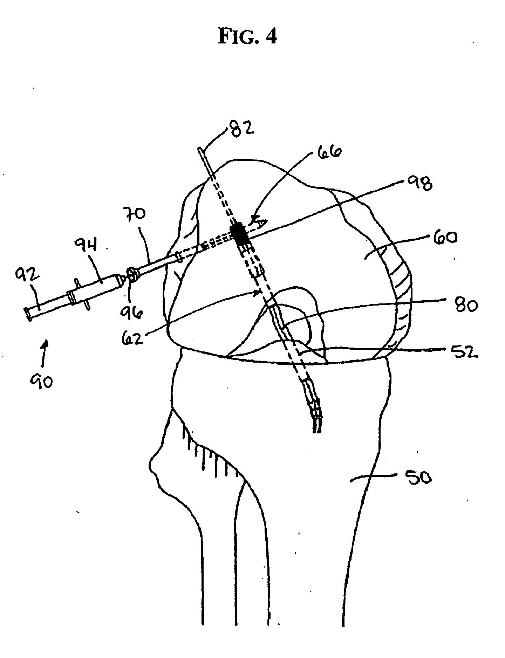

As shown in FIG. 4, once the ligament graft 80 is positioned within the femoral tunnel 62 the surgeon can inject an adhesive 98 into the femoral tunnel 62 to secure the ligament graft 80 therein. While the adhesive 98 can be injected through any of the tunnels using a variety of techniques, in the illustrated exemplary embodiment the adhesive 98 is injected through the transverse tunnel 66 using a syringe 90. In particular, the syringe 90 is placed through the sleeve 70 in the transverse tunnel 66 and into the femoral tunnel 62, and a plunger 92 on the syringe 90 is then moved into a barrel 94 to eject the adhesive 98 from the barrel 94, through the needle 96, and into the femoral tunnel 62. The amount of adhesive 98 can vary, but in an exemplary embodiment, the adhesive 98 fills a terminal end portion of the femoral tunnel 62 adjacent to the end surface 62e.

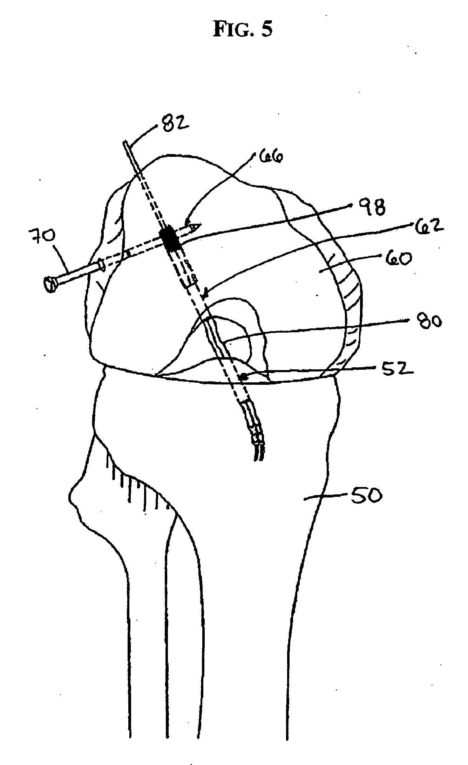

Once the terminal end portion of the femoral tunnel 62 is filled with the adhesive 98, the suture 82 is further tensioned to pull the ligament graft 80 into the terminal end portion such that the ligament graft 80 abuts the end wall 62e of the femoral tunnel 62, as shown in FIG. 5. As a result, the ligament graft 80 is brought into contact with the adhesive 98, which is spread in and around the ligament graft 80. The adhesive 98 thus extends between the end surface 62e and the ligament graft 80, and forms a cap that surrounds the ligament graft 80, and optionally that extends into portions of the ligament graft 80. In other embodiments, the ligament graft 80 can be fully pulled into the femoral tunnel 62 and into contact with the end surface 62e, and then the adhesive 98 can be injected into the tunnel 62 until it completely surrounds and encapsulates an end portion of the ligament graft 80.

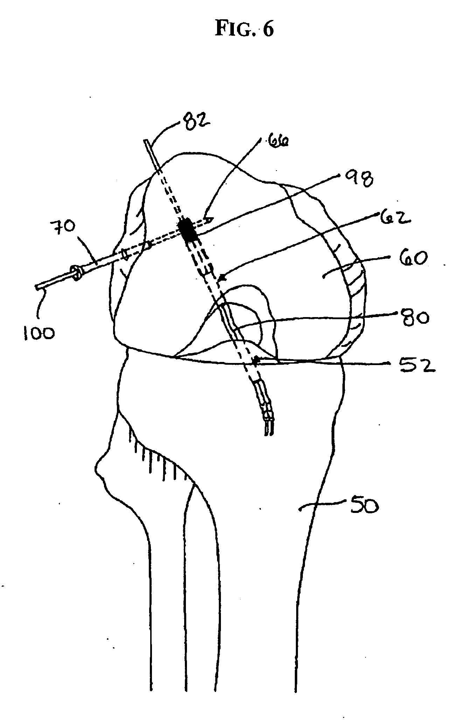

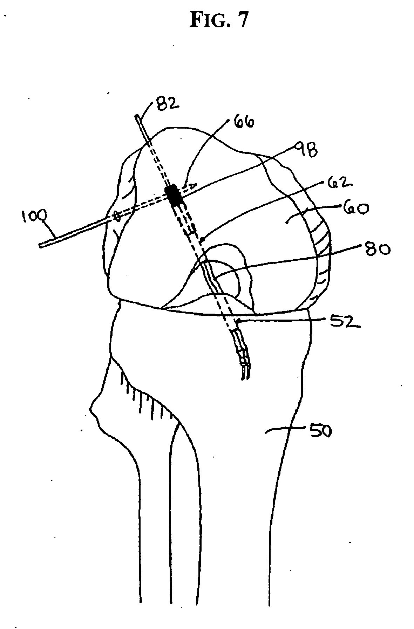

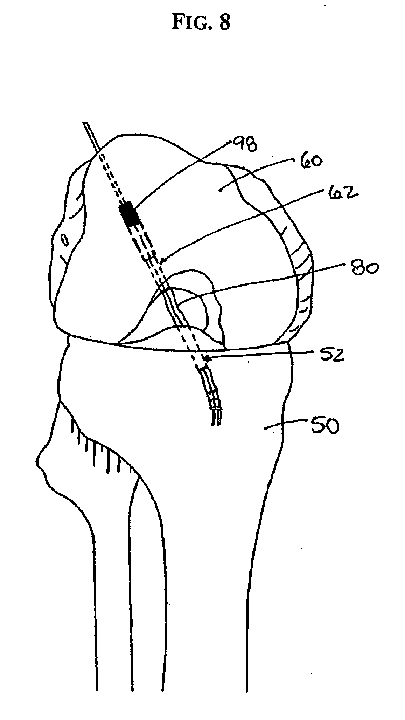

Since the adhesive 98 can take time to cure, the first end of the ligament graft 80 can be temporarily secured in the femoral tunnel 62 using a cross-pin. FIG. 6 illustrates one exemplary embodiment of a cross-pin 100 that is used to secure the ligament graft 80 in the femoral tunnel 62. As shown, the cross-pin 100 is in the form of an elongate member that is inserted through the sleeve 70 in the transverse tunnel and through a loop formed in the terminal end of the ligament graft 80. The cross-pin 100 can also extend across the femoral tunnel 62 and into a further portion of the transverse tunnel. In an exemplary embodiment, the cross-pin 100 has a length that allows a portion of the cross-pin 100 to remain outside of the sleeve 70 while the remainder of the cross-pin 100 extends through the transverse tunnel 66, through the graft 80, and completely across the femoral tunnel 62. Once the ligament graft 80 is temporarily secured within the femoral tunnel 62 using the cross-pin 100, the sleeve 70 can be removed leaving the cross-pin 100 in place, as shown in FIG. 7. The second end of the ligament graft 80 can also be tensioned and secured within the tibial tunnel 52 using the same anchoring technique, or using other anchoring techniques known in the art. When the adhesive 98 is finally cured, the cross-pin 100 can be removed leaving the ligament graft fixed within the femoral tunnel 62, as shown in FIG. 8.

A person skilled in the art will appreciate that the cross-pin 100 can have a variety of configurations. The cross-pin can also be formed from a variety of materials, but in accordance with the invention it is preferably formed from or coated with a material that prevents adhesion to the adhesive. For example, the fixation device can be formed from or coated with a fluoropolymer plastic resin, such as Teflon®. A person skilled in the art will appreciate that a variety of other materials can be used to prevent adhesive between the cross-pin and the adhesive. The sleeve 70 can also optionally be formed from or coated with a protective material that prevents adhesive to the adhesive.

The materials used to form the adhesive can also vary, and virtually any bone glue or cement known in the art can be used. Since a cross-pin is temporarily used to maintain the ligament graft within the femoral tunnel while the adhesive dries, the drying time for the adhesive can significantly vary and the surgeon has more freedom to select a desired adhesive. The adhesive can also be bioabsorbable or non-absorbable. In certain exemplary embodiments, the adhesive preferably has a viscosity that allows it to be injected into the femoral tunnel without the adhesive dripping, but that allows it to spread around the ligament graft as the graft is pulled fully into the femoral tunnel. By way of non-limiting example, exemplary adhesives include bone glues, such as biocompatible bone glues including 2-octyl cyanoacrylate and the like and equivalent thereof, bone cements, such as conventional biocompatible bone cements including polymethylmethacrylate and the like, as well as dental implant cements such as Premiere Dental Implant Cement®.

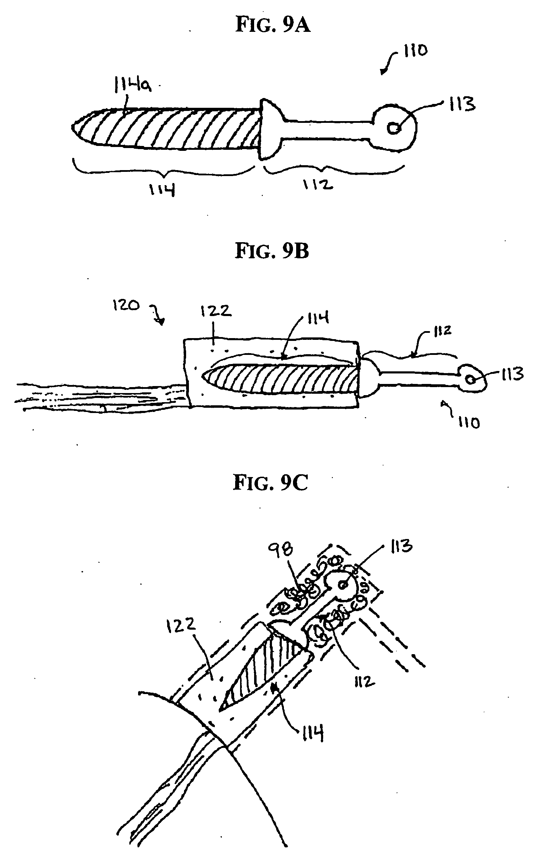

As previously indicated, the ligament graft can also have a variety of configurations, and it can be directly affixed within the bone tunnel, or an anchoring element can be used to affix the ligament graft within the bone tunnel. In the embodiments shown in FIGS. 9A-11C, each ligament graft is in the form of a bone-to-bone graft that is mated to an anchoring element having surface features that facilitate adhesion thereof within a bone tunnel.

As indicated above, in the embodiment shown in FIGS. 9A-9C, an anchoring element 110 can be used to affix a bone-to-bone graft 120 within a bone tunnel. While the anchoring element 110 can have a variety of configurations, in one exemplary embodiment it preferably includes a first portion 112 that is adapted to be adhesively mated to a bone tunnel, and a second portion 114 that extends from the first portion 112 and that is adapted to mate to a bone graft 122 of a bone-to-bone graft 120.

The first portion 112 can have a variety of configurations, and various techniques can be used to facilitate adhesion between the first portion 112 and a bone tunnel. In the illustrated embodiment, the first portion 112 is in the form of a post having an eyelet formed on a terminal end thereof. The eyelet includes an opening 113 formed therein for receiving a suture which can be used to pull the anchor 110 and ligament graft 120 attached thereto into a bone tunnel. The eyelet, as well as the post, can also facilitate adhesion of the first portion 112 to a bone tunnel, as an adhesive will surround the post and extend into the eyelet, as shown in FIG. 9C. A person skilled in the art will appreciate that the first portion can have a variety of other shapes, and it can include a variety of other features formed thereon, such as grooves, bores, protrusions, etc.

The second portion 114 of the anchor 110 can also have a variety of configurations, and various techniques can be used to mate the second portion 114 to a bone graft 122. As shown in FIGS. 9A-9C, the second portion 114 includes threads 114a formed thereon for allowing the second portion 114 to be threaded into the bone graft 120, as shown in FIG. 9B. Other mating techniques, including adhesives, can be used to mate the second portion 114 to the bone graft 122.

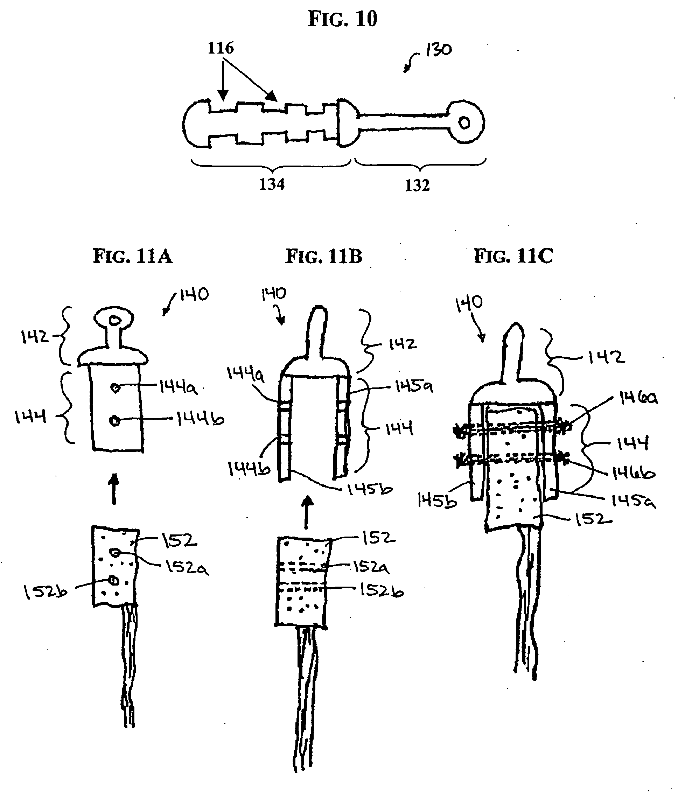

FIG. 10 illustrates another embodiment of an anchor 130 that is adapted to mate to a bone graft, and that is adapted to be affixed within a bone tunnel using an adhesive. The anchor 130 is similar to the anchor 110 shown in FIG. 9A as it includes a first portion 132 having a post and an eyelet, and a second portion 134 adapted to mate to a bone graft. In this embodiment, however, the second portion 134 includes annular cut-out regions 116 formed along a length thereof for receiving an adhesive. The adhesive can be injected into and around the cut-out regions 116 and the second portion 134 can be inserted into a bore drilled in a bone graft to adhere the second portion 134 to the bone graft.

FIGS. 11A-11C illustrate yet another embodiment of an anchor 140 adapted to mate to a bone graft 152 of a bone-to-bone ligament graft 50, and to be adhered within a bone tunnel. Similar to the embodiments shown in FIGS. 9A-10, the anchor 140 includes a first portion 142 having a post and an eyelet formed thereon, and a second portion 144 that is adapted to mate to a bone graft. In this embodiment, the second portion 144 includes opposed arms 145a, 145b that are adapted to receive a bone graft 152 therebetween. While the arms 145a, 145b can optionally be adhesively mated to the bone graft 152, the arms 145a, 145b can also or alternatively be sutured to the bone graft 152. Thus, the opposed arms 145a, 145b can each include one or more thru-bores formed therein. FIGS. 11A-11C illustrate two thru-bores 144a, 144b formed in each arm 145a, 145b. Two corresponding thru-bores 152a, 152b can also be drilled through the bone graft 152 to allow suture 146a, 146b to extend through the bone graft 152 to attach the bone graft 152 to the arms 145a, 145b, as shown in FIG. 11C. A person skilled in the art will appreciate that various other techniques can be used to mate the bone graft 152 to the arms 145a, 145b, and that the arms 145a, 145b can have a variety of other configurations. For example, the arms 145a, 145b can threadably engage the bone graft 152.

In use, the anchoring elements 110, 130, 140 of FIGS. 9A-11C can be implanted within a bone tunnel using various methods known in the art. In an exemplary embodiment, however, the anchors 110, 130, 140 are affixed within a bone tunnel using the exemplary methods previously described herein. In particular, after the anchoring element 110, 130, 140 is attached to a bone graft, the anchoring element 110, 130, 140 can be pulled through the tibial tunnel and at least partially into the femoral tunnel. This can be achieved using various techniques, for example, by tensioning a suture that is attached to the eyelet of the anchor. An adhesive, e.g., adhesive 98 as shown in FIG. 9C, can then be introduced into the femoral tunnel, preferably via the transverse tunnel, to fill a terminal end portion of the femoral tunnel. The anchor 110, 130, 140 can be further pulled into the femoral tunnel, causing the adhesive to spread around the first portion of the anchor 110, 130, 140. Alternatively, the anchor 110, 130, 140 can be fully pulled up into the femoral tunnel prior to introducing the adhesive. A cross-pin is inserted through the hole in the eyelet to maintain the anchor 110, 130, 140, and thus the ligament graft, within the femoral tunnel until the adhesive cures.

One of ordinary skill in the art will appreciate further features and advantages of the invention based on the above-described embodiments. Accordingly, the invention is not to be limited by what has been particularly shown and described, except as indicated by the appended claims.

## Claims

1. A device for ligament reconstruction, comprising:
ligament graft (80; 120) suitable for insertion at least partially into a first bone tunnel (62) drilled into bone, comprising:
a bone graft (122; 152) having a ligament extending therefrom; and
an anchor (110) having a first portion (112) having an eyelet having an opening (113) extending therethrough (113) adapted for receiving a cross-pin (100), and a second portion (114) mated to the bone graft,
a cross-pin (100) suitable for insertion through a transverse tunnel (52) formed in bone and intersecting the first bone tunnel (62) and adapted for insertion through the opening (113) in the eyelet for maintaining the ligament graft (80) in said bone tunnel (62)
**characterised in that**,
the device further comprises an adhesive (98), wherein the opening (113) of the eyelet is adapted to receive the adhesive (98) for adhesively mating the first portion to the first bone tunnel (62); and
the cross-pin (100) is adapted to prevent adhesion between the adhesive (98) and the cross-pin (100) such that the cross-pin can maintain the ligament graft (80) in said bone tunnel (62) to allow said adhesive to cure and be removed when the adhesive is cured.

2. The device of claim 1, wherein the second portion (114) comprises an elongate member having threads (114a) formed thereon for engaging the bone graft (122).

3. The device of claim 1, wherein the second portion (114) includes opposed arms (145a, 145b) for receiving the bone graft (152) therebetween.

4. The device of claim 3, wherein the opposed arms (145a, 145b) and the bone graft (152) include at least one suture hole (144a, 144b, 152a, 152b) extending therethrough for receiving a suture (146a, 146b) adapted to mate the bone graft to the opposed arms.

## Patentansprüche

1. Vorrichtung zur Bänderrekonstruktion mit
einem Bändertransplantat (80; 120), das dazu geeignet ist, mindestens teilweise in einen ersten in Knochen gebohrten Knochentunnel (62) eingeführt zu werden, mit
einem Knochentransplantat (122; 152), von dem sich ein Band erstreckt, und
einem Anker (110) mit einem ersten Abschnitt (112) mit einer Öse, durch die sich eine Öffnung (113) erstreckt, die zur Aufnahme eines Kreuzzapfens (100) geeignet ist, und einem zweiten Abschnitt (114), der mit dem Knochentransplantat zusammenpasst,
einem Kreuzzapfen (100), der zum Einführen durch einen Quertunnel (52), der im Knochen ausgebildet ist und den ersten Knochentunnel (62) schneidet, sowie zum Einführen durch die Öffnung (113) in der Öse geeignet ist, um das Bändertransplantat (80) im Knochentunnel (62) zu halten,
**dadurch gekennzeichnet, dass**
die Vorrichtung ferner einen Klebstoff (98) umfasst, wobei die Öffnung (113) der Öse dazu geeignet ist, den Klebstoff (98) aufzunehmen, um den ersten Abschnitt passend mit dem ersten Knochentunnel (62) zusammenzukleben, und
der Kreuzzapfen (100) dazu geeignet ist, Haftung zwischen dem Klebstoff (98) und dem Kreuzzapfen (100) zu verhindern, so dass der Kreuzzapfen das Bändertransplantat (80) im Knochentunnel (62) halten kann, damit der Klebstoff aushärten kann, und nach dem Aushärten des Klebstoffs entfernt werden kann.

2. Vorrichtung nach Anspruch 1, wobei der zweite Abschnitt (114) ein längliches Element mit einem daran ausgebildeten Gewinde (114a) zur Ineingriffnahme des Knochentransplantats (122) umfasst.

3. Vorrichtung nach Anspruch 1, wobei der zweite Abschnitt (114) gegenüberliegende Arme (145a, 145b) aufweist, die zwischen sich das Knochentransplantat (152) aufnehmen.

4. Vorrichtung nach Anspruch 3, wobei die gegenüberliegenden Arme (145a, 145b) und das Knochentransplantat (152) mindestens einen sich dort hindurch erstreckenden Stichkanal (144a, 144b, 152a, 152b) zur Aufnahme einer Naht (146a, 146b) aufweisen, die dazu geeignet ist, das Knochentransplantat mit den gegenüberliegenden Armen zusammenzupassen.

## Revendications

1. Dispositif pour une reconstruction ligamentaire, comportant :
une greffe (80 ; 120) ligamentaire pouvant être introduite au moins partiellement dans un premier tunnel (62) osseux percé dans l'os, comportant :
une greffe (122 ; 152) osseuse présentant un ligament s'étendant depuis cette dernière ; et
un ancrage (110) présentant une première section (112) munie d'un oeillet présentant une ouverture (113) traversant ce dernier (113) conçu pour recevoir une contre-goupille (100), et une seconde section (114) appariée à la greffe osseuse,
une contre-goupille (100) pouvant être introduite dans un tunnel (52) transversal formé dans l'os et croisant le premier tunnel (62) osseux et conçue pour être introduite dans l'ouverture (113) de l'oeillet afin de maintenir la greffe (80) ligamentaire dans ledit tunnel (62) osseux.
**caractérisé en ce que**,
le dispositif comporte en outre un adhésif (98), dans lequel l'ouverture (113) de l'oeillet est conçue pour recevoir l'adhésif (98) afin d'apparier la première section au premier tunnel (62) osseux ; et
la contre-goupille (100) est conçue pour empêcher l'adhésif (98) et la contre-goupille (100) d'adhérer l'un à l'autre de sorte que la contre-goupille peut maintenir la greffe (80) ligamentaire dans ledit tunnel (62) osseux et être enlevée lorsque l'adhésif a durci.

2. Dispositif selon la revendication 1, dans lequel la seconde section (114) comporte un élément oblong muni de fils (114a) formés sur ce dernier pour se mettre en prise avec la greffe (122) osseuse.

3. Dispositif selon la revendication 1, dans lequel la seconde section (114) comprend des branches (145a, 145b) opposées pour recevoir la greffe (152) osseuse entre ces dernières.

4. Dispositif selon la revendication 3, dans lequel les branches (145a, 145b) opposées et la greffe (152) osseuse comprennent au moins un trou (144a, 144b, 152a, 152b) de suture les traversant pour recevoir une suture (146a, 146b) conçue pour apparier la greffe osseuse aux branches opposées.
